# EUROPÄISCHE PATENTANMELDUNG

(11) **EP 1 426 061 A2**
(43) Veröffentlichungstag der Anmeldung: **09.06.2004**
(21) Anmeldenummer: 03090424.7
(22) Anmeldetag: 04.12.2003
(51) Int. Cl.: A61K 47/48

(54) **Kosmetische Zusammensetzung mit elektrischen Ladungsträgern**

(30) Priorität: 04.12.2002 DE 10257240
(71) Anmelder: Coty B.V., 2031 CC Haarlem (NL)
(72) Erfinder: Golz-Berner, Karin, 98000 Monaco (MC); Walzel, Bernd, 8003 Zürich (CH); Zastrow, Leonhard, 98000 Monaco (MC)
(74) Vertreter: Walter, Wolf-Jürgen

(57) **Zusammenfassung**

Die Erfindung betrifft eine kosmetische Zusammensetzung, die elektrische Ladungsträger neben bestimmten Wirk- und Hilfsstoffen enthält. Erfindungsgemäß enthält die Zusammensetzung 0,1 bis 10 Gew-% eines kosmetisch annehmbaren Elektret-Feststoffes mit einer Teilchengröße von 0,05 bis 100 µm, der als Elektret ein induziertes permanentes Dipolmoment aufweist und ein permanentes elektrischen Dipoleld mit einer Feldstärke von 500 bis 10⁷ Vm⁻¹ hat, sowie kosmetische Trägerstoffe, Hilfsstoffe, weitere Wirkstoffe oder ein Gemisch davon mit einem Anteil bis 100 Gew-%.

## Beschreibung

Die Erfindung betrifft eine kosmetische Zusammensetzung, die elektrische Ladungsträger neben bestimmten Wirk- und Hilfsstoffen enthält.

Aus der DE 43 25 071 sind u.a. auch kosmetische Präparate bekannt, die permanentmagnetische Einkristalle enthalten und die die Mikrozirkulation in der Haut anregen sollen.

Die WO 99/18892 beschreibt Biopolymere, insbesondere alphahelicale Polypeptide mit Elektreteigenschaften, die für ein verbessertes Gewebewachstum oder für Transplantationen eingesetzt werden können.

Es ist auch bekannt, als Filtermaterialien bei der Luftreinigung bestimmte elektrisch polarisierte Materialien einzusetzen.

Der Erfindung liegt die Aufgabe zugrunde, die Aufnahmefähigkeit von Hautzellen für die Zuführung von Nähr- und Wirkstoffen zu verbessern.

Erfindungsgemäß ist die kosmetische Zusammensetzung mit elektrischen Ladungsträgern dadurch gekennzeichnet, daß sie 0,1 bis 10 Gew-% eines kosmetisch annehmbaren Elektret-Feststoffes mit einer Teilchengröße von 0,05 bis 100 im enthält, der als Elektret ein induziertes permanentes Dipolmoment aufweist und ein permanentes elektrischen Dipol-Feld mit einer Feldstärke von 500 bis 10⁷ Vm⁻¹ hat, und daß sie weiterhin kosmetische Trägerstoffe, Hilfsstoffe, weitere Wirkstoffe oder ein Gemisch davon mit einem Anteil bis 100 Gew-% enthält, wobei die Prozentangaben auf das Gesamtgewicht der Zusammensetzung bezogen sind.

Bevorzugte Elektrete haben ein Dipolfeld mit einer Feldstärke von 10⁵ bis 10⁷ Vm⁻¹.

Unter Elektreten werden allgemein Stoffe verstanden, die eine permanente entgegengesetzte elektrische Ladung an zwei gegenüberliegenden Flächen aufweisen. Damit bilden sie einen permanenten elektrischen Dipol, der von einem elektrischen Feld umgeben ist (Dipolfeld). Bestimmte natürliche Elektrete wie Turmalin sind bereits seit langem bekannt. Die Elektreteigenschaften können jedoch einer Reihe von Stoffen auch durch Einwirkung von außen verliehen werden. Dies kann thermisch erfolgen, indem der Stoff über die Curie-Temperatur erhitzt wird und in diesem Zustand einem elektrischen Feld ausgesetzt wird. Die sich im Feld orientierenden Dipole werden durch Abkühlung eingefroren.
Der dadurch erreichte Nichtgleichgewichtszustand geht nach einer bestimmten Relaxationszeit, die bei Elektreten im Bereich von Jahren liegt, wieder in einen Gleichgewichtszustand über. Die gleichen Zustände können auch photoelektrisch herbeigeführt werden.

Die Messung der Feldstärken von Elektreten erfolgt üblicherweise mit hochohmigen Voltmetern über eine einfache Spannungsmessung der Kompensationsspannung und bei gleichzeitiger Erfassung des Abstandes zwischen dem Material und der Elektrode und in Abhängigkeit von der Anzahl der dazwischenliegenden Teilchen.

Sowohl organische als auch anorganische Materialien können in den Elektretzustand überführt werden. Dabei kann die Polarisation eine atomare Polarisation, eine Dipolpolarisation, eine Grenzflächenpolarisation, eine Raumladungspolarisation oder eine Polarisation durch Ladungsübertragung sein. Bei der in diesem Zusammenhang hauptsächlich zu berücksichtigenden Dipolpolarisation werden beispielsweise bestimmte Polymere, wie polymerisierte Fluorcarbone, oder Gläser, Keramiken oder Glaskeramiken in den Elektretzustand überführt durch Erhitzen, Aussetzen des Materials einem elektrischen Feld und Abkühlen, wobei das erzielte Dipolmoment auch beim nachfolgenden Zerkleinern des Materials erhalten bleibt.

Das dadurch diesen Materialien verliehene Dipolmoment, das hier als "induziertes permanentes elektrisches Dipolmoment" bezeichnet werden soll, liegt deutlich über dem bekannten Dipolmoment von Stoffen, die nicht induziert worden sind. Das Dipolmoment für viele Verbindungen ist bekannt (z.B. Handbook of Chemistry and Physics, 70. Aufl., Teil E, S. 59 ff.; oder McClellan, Tables of Experimental Dipole Moments, San Francisco: Freeman 1963) oder kann z.B. durch Messung der Dielektrizitätskonstante und der Dichte bei verschiedenen Temperaturen und mit Hilfe der Debye-Clausius-Mosotti-Gleichung ermittelt werden.

Die erfindungsgemäßen Feststoffe, die in den Elektretzustand überführt worden sind, haben ein permanentes elektrisches Dipolmoment von etwa 10⁻¹⁵ bis 10⁻²⁴ C.m (Coulomb mal Meter) bei der genannten Feldstärke und Teilchengröße. Feldstärke und Dipolmoment zeigen ihre Wirkung nur in Wechselwirkung auf Zellebene und nicht nach außen in die Umgebung der kosmetischen Zusammensetzung.

Die Zerkleinerung des elektretischen Materials erfolgt für die kosmetische Anwendung bis zu einer Teilchengröße von 0,05 bis 100 µm, vorzugsweise 3 bis 80 µm.

Als Elektretmaterialien bevorzugt sind polymerisierte Fluorcarbone, die aus der Gruppe ausgewählt sind, bestehend aus Polytetrafluorethylen (PTFE), Fluorethylenpropylen (FEP), Polyvinylidenfluorid (PVDF), amorphes Fluorpolymer (AF) und Gemische davon.

Als Keramiken oder Glaskeramiken sind solche mit oxidischen Grundstoffen bevorzugt, ausgewählt aus der Gruppe, bestehend aus Zirkonoxid, Titanoxid, Magnesiumoxid, Lithiumoxid, Calciumoxid, Siliciumdioxid, Bariumoxid und Gemischen davon.

Es wurde gefunden, dass die erfindungsgemäße kosmetische Zusammensetzung die Aufnahmefähigkeit von Hautzellen für die Zuführung von Nähr- und Wirkstoffen deutlich verbessert.

Ein bevorzugter Bereich für das Elektretmaterial in der kosmetischen Zusammensetzung ist 0,1-8 Gew-%, insbesondere 0,1-5 Gew-%.

In einer bevorzugten Ausführungsform der Erfindung enthält die kosmetische Zusammensetzung das Elektretmaterial, vorzugsweise Polytetrafluorethylen, und wenigstens 0,1 Gew-%, vorteilhaft 0,5-2 Gew-%, bezogen auf die Gesamtzusammensetzung, Vitamin A oder eine Vitamin A-Verbindung. In Vergleichsversuchen wurden gefunden, daß in Anwesenheit des Elektretmaterials die Vitamin A-Aufnahme durch die Haut im gleichen Zeitraum von 0-4 Stunden um etwa 40 bis 70 % verbessert wird.

In einer weiteren bevorzugten Ausführungsform der Erfindung wurde eine ähnliche verbesserte Wirkstoffaufnahme bei 0,1-2 Gew-% Vitamin E und Derivaten davon gefunden, bei dem die Aufnahme durch die Haut um etwa 30-50 % verbessert wird.

In einer weiteren bevorzugten Ausführungsform der Erfindung wurde eine ähnliche verbesserte Wirkstoffaufnahme bei 0,1-3 Gew-% Kreatin gefunden, bei dem die Aufnahme durch die Haut um etwa 25-55 % verbessert wird.

Auch die Aufnahme von 0,1-3 Gew-% Vitamin B wurde verbessert.

Bevorzugte Vitaminderivate sind z.B. Tocopherylacetat, Tocopherylpalmitat, Tocopherylsuccinat, Tocopherylpropionat, Tocopheryloleat, Tocopheryllinolat, Tocopherylsorbat sowie Retinylacetat, Retinylpalmitat, Retinylbutyrat, Retinylpropionat, Retinyl-octanoat, Retinyllaurat, Retinyloleat, Retinyllinolat.

Vorteilhaft für die erfindungsgemäßen Zubereitungen kann es sein, dass sie zusätzlich asymmetrische lamellare Aggregate enthalten, wobei diese Aggregate aus Phospholipiden und mit Sauerstoff beladenem Fluorcarbon oder einem Fluorcarbongemisch bestehen und deren Gehalt an Fluorcarbon im Bereich von 0,2 bis 100 % Gewicht/Volumen liegt, wobei das Phospholipid einen Phosphatidylcholingehalt von mehr als 30 bis 99 Gewichts-% hat, und wobei diese Aggregate eine Hautpenetrierung in Abhängigkeit von der kritischen Löslichkeitstemperatur der Fluorcarbone besitzen. Diese Aggregate sind Sauerstoffträger und ermöglichen ein Penetrieren des Sauerstoffs in die Haut und damit eine bessere Versorgung der Haut mit Sauerstoff (für den Einsatz asymmetrischer lamellarer Aggregate s.a. DE-B-42 21 255 oder WO94/00098).

Einen weiteren Wirkstoff, den das erfindungsgemäße Kosmetikum enthalten kann, sind fein verteilte hartmagnetische Einbereichsteilchen (Einkristalle) mit hoher Koerzitivkraft von 3000 bis 5000 Oerstedt und mit Korngrößen im Bereich von 50 bis 1200 nm, vorzugsweise 50-250 nm, mit oder ohne die o.g. asymmetrischen lamellaren Aggregate, wobei diese hartmagnetischen Teilchen insbesondere Barium- und/oder Strontiumhexaferrite sind, erzeugt nach der Glaskristallisationstechnik durch Züchtung von Einkristallen aus einer abgeschreckten Glasschmelze (siehe WO95/03061 z.B. Beispiel 2, 3 oder 7; und WO98/44895 z.B. Beispiel 1C). Diese Einkristalle erhöhen die Mikrozirkulation und verstärken somit noch den durch die erfindungsgemäßen Teilchen hervorgerufenen Effekt.

Die erfindungsgemäße Zusammensetzung kann vorteilhaft weitere kosmetische Wirkstoffe enthalten. Dazu gehören z. B. Emulgatoren, anorganische und organische Lichtschutzmittel, Radikalfänger, Feuchthaltemittel, Vitamine, Enzyme, pflanzliche Wirkstoffe, Polymere, Antioxidationsmittel, entzündungswidrige natürliche Wirkstoffe; Aufschlußprodukte von Hefen oder pflanzlichen Stoffen, hergestellt durch ein schonendes Ultraschall-Aufschlußverfahren gemäß WO 94/13783; Kaolin sowie mit SiO₂ modifiziertes Kaolin gemäß WO94/17588.

Ein bevorzugter weiterer Zusatz für das erfindungsgemäße Kosmetikum ist eine Wirkstoffzubereitung mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser (z.B. WO99/66881 Wirkstoffkomplex gemäß Beispiel 1 oder 2; WO 01/26617 Wirkstoffkomplex gemäß Beispiel 1).

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Ebenfalls einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch eingesetzt werden können.

Besonders bevorzugte zusätzliche Wirkstoffe sind mit Sauerstoff beladene asymmetrische lamellare Aggregate gemäß WO 94/00109 oder WO94/00098 und hartmagnetische Einkristalle gemäß WO 95/03061.

Das erfindungsgemäße Präparat enthält weiterhin kosmetische Hilfs- und Trägerstoffe, wie sie üblicherweise in solchen Zubereitungen verwendet werden, z.B. Wasser, Konservierungsmittel, Farbstoffe, Pigmente mit färbender Wirkung, Verdikungsmittel, Duftstoffe, Alkohole, Polyole, Ester, Elektrolyte, Gelbildner, polare und unpolare Öle, Polymere, Copolymere, Emulgatoren, Wachse, Stabilisatoren.

Die für die Erfindung eingesetzten Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Polyole sind z.B Propylenglycol, Dipropylenglycol, Ethylenglycol, Isoprenglycol, Glycerin, Butylenglycole, Sorbitol und Gemische davon. Der Anteil des Polyols liegt im Bereich von 0,1 bis etwa 20 Gew-% der Gesamtzusammensetzung.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure usw.

Zu geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate, Polyvinylalkohol, Polyvinylpyrrolidon (PVP), Montmorillonit. Bevorzugt sind quaternisierter Guar, Carbomer und PVP.

Die Verwendung der erfindungsgemäßen kosmetischen Zusammensetzungen kann z.B. erfolgen in Form von Sonnencremes, Sonnengelen, After-sun-Produkten, Tagescremes, Nachtcremes, Masken, Körperlotionen, Reinigungsmilch, Körperpuder, Augenkosmetik, Haarmasken, Haarspülungen, Haarshampoos, Duschgelen, Duschölen, Badeölen und in Produkten der dekorativen Kosmetik wie
Deo-Stiften, Parfüm-Stiften, Lippenstiften, Gelen, Lidschattens, Kompaktprodukte wie Kompaktpuder oder Kompaktwachs, Rouge, Grundierung, Make-up usw. Die Herstellung derartiger Produkte erfolgt auf eine Weise, wie sie dem Fachmann auf diesem Gebiet bekannt ist.

An die Einarbeitung der Elektrete in die kosmetischen Formulierungen sind keine besonderen Bedingungen geknüpft. Es ist vorteilhaft, die Elektretteilchen zuletzt und nicht bei höheren Temperaturen oberhalb 50 EC einzumischen.

Ein weiterer Gegenstand der Erfindung besteht in der Verwendung von Elektreten mit einer Feldstärke von 500 bis 10⁷ Vm⁻¹ des Dipolfeldes in einem Anteil von 0,1 bis 10 Gew-% und mit einer Teilchengröße von 0,05 bis 100 µm, ausgewählt unter polymerisierten Fluorcarbonen, Polyethylenterephthalat, Polymethylmethacrylat, Polyimiden, Polypropylen, Polyethylen, Polyurethanen, Polyharnstoffen, Keramiken, Gläsern, Glaskeramiken und Gemischen davon, die jeweils in einen induzierten Elektretzustand überführt worden sind, zusammen mit kosmetischen Trägerstoffen, Hilfsstoffen, weiteren Wirkstoffen oder einem Gemisch davon mit einem Anteil bis 100 Gew-% in kosmetischen Cremes, Lotionen, Gelen, Pudern und Stiften.

Ein weiterer Gegenstand der Erfindung besteht in der Bereitstellung einer kosmetischen Zusammensetzung mit elektrischen Ladungsträgern, dadurch gekennzeichnet, dass die Zusammensetzung 0,1 bis 10 Gew-% eines kosmetisch annehmbaren Elektret-Feststoffes mit einer Teilchengröße von 0,05 bis 100 µm enthält, der als Elektret ein induziertes permanentes Dipolmoment aufweist und ein permanentes elektrischen Dipolfeld mit einer Feldstärke von 500 bis 10⁷ Vm⁻¹ hat, sowie kosmetische Trägerstoffe, Hilfsstoffe, weitere Wirkstoffe oder ein Gemisch davon mit einem Anteil bis 100 Gew-% enthält, wobei die Prozentangaben auf das Gesamtgewicht der Zusammensetzung bezogen sind,
erhältlich durch Erhitzen eines nicht-ferromagnetischen Feststoffes auf eine Temperatur unterhalb seiner Schmelztemperatur, Aussetzen des Feststoffes einem elektrischen Feld von 1000 bis 10⁷ V/m, spontane Abkühlung des Feststoffes, Mahlen des hergestellten Elektret-Feststoffes auf eine Teilchengröße von 0,05 bis 100 µm und Einmischen in eine kosmetische Zusammensetzung unterhalb 50 °C.

Bei Einsatz von Polymeren als nicht-ferromagnetische Materialien sind Temperaturen oberhalb des Erweichungspunktes bevorzugt, um das Elektretmaterial als Folie herstellen zu können, um Oberflächenladungsdichten von bis zu 10⁻⁷ C/cm² auf der Folienoberfläche zu erreichen.

Die Erfindung soll nachstehend durch Beispiele näher erläutert werden. Alle Angaben erfolgen in Gewichtsprozent, sofern nichts anderes angegeben ist.

### Beispiel 1 Nachtcreme

| **Phase A** | |
|---|---|
| Wasser | q.s. ad 100 |
| Glycerine | 2,0 |
| Propylenglycol | 2,0 |
| Carbomere | 0,3 |

| **Phase B** | |
|---|---|
| Glyceryl Stearate | 1,0 |
| C12-15 Alkyl Benzoate | 1,5 |
| Silicone-Dimethicone | 2,5 |

| **Phase C** | |
|---|---|
| Triethanolamin | 0,3 |

| **Phase D** | |
|---|---|
| Polytetrafluorethylene 10-60 µm | 5,0 |
| Vitamin C | 1,0 |
| Vitamin B6 | 2,0 |
| Parfüm | 0,5 |
| Konservierungsmittel | 0,5 |

Die Phasen A und B werden nach separater Herstellung unter Rühren auf etwa 75 °C erwärmt, zusammengegeben und 20 Minuten bei ca. 3000 U/min homogenisiert. Danach wird die Phase C zugegeben, 5 Minuten homogenisiert und aus etwa 45 °C abgekühlt. Unter weiterem Abkühlen und Rühren wird die Phase D zugegeben und bei 30 °C für 20 Minuten homogenisiert.
Das PTFE hatte ein Dipolmoment von etwa 10⁻¹⁹ C.m .

### Beispiel 2 Fluid für den Tag

| **Phase A** | |
|---|---|
| Cetyl Palmitate | 1,0 |
| Cetearyl Isononanoate | 1,8 |
| Squalane | 2,9 |

| **Phase B** | |
|---|---|
| Wasser | q.s. ad 100 |
| Sodium Citrate | 0,1 |
| Glycerin | 2,0 |
| Polyacrylamide | 0,1 |

| **Phase C** | |
|---|---|
| Fluorcarbonpolymer-Gemisch¹ | 2,0 |
| Konservierungsmittel | 0,5 |
| Parfüm | 0,2 |
| Kaffein | 0,3 |

| | |
|---|---|
| ¹ Gemisch aus 20% Polytetrafluorethylen, 30% Fluorethylenpropylen, 30% Polyvinylidenfluorid und 20% amorphes Fluorpolymer (Teilchengröße 50-90 µm, Dipolmoment etwa 10⁻¹⁶ C.m). | |

Die weitere Herstellung der kosmetischen Zusammensetzung erfolgt entsprechend Beispiel 1.

### Beispiel 3 Bodylotion I

| **Phase A** | |
|---|---|
| PEG 100 Stearate | 3,5 |
| C12-15 Alkyl Benzoate | 1,0 |
| Shea butter fruit | 2,5 |

| **Phase B** | |
|---|---|
| Wasser | q.s. ad 100 |
| Carbomer | 0,2 |
| Glycerin | 2,0 |

| **Phase C** | |
|---|---|
| Triethanolamin | 0,2 |

| **Phase D** | |
|---|---|
| Polytetrafluorethylen 0,5-40 µm | 0,15 |
| Vitamin A | 0,1 |
| Parfüm | 0,8 |
| Konservierungsmittel | 0,5 |

### Beispiel 4 und 5 Bodylotion II und III

Es wurde eine Lotion gemäß Beispiel 3 hergestellt mit Ausnahme dessen, dass anstelle von Vitamin A 0,5 % eines Wirkstoffkomplexes gemäß Beispiel 1 der WO 01/26617 eingesetzt wurde, bestehend aus Rindenextrakt von Quebracho blanco, Seidenraupenextrakt, Gel, Phospholipiden, Superoxid-Dismutase aus Hefe-Aufschlußprodukt, Cyclodextrinen und Wasser (Beispiel 4).

Weiterhin wurde neben 0,1% Vitamin A der genannte Wirkstoffkomplex mit einem Anteil von 0,5% als Bodylotion III hergestellt (Beispiel 5). Dipolmomente etwa 10⁻²¹* C.m.

### Beispiel 6 Nacht- und Tagescreme I

| **Phase A** | |
|---|---|
| Wasser | q.s. ad 100 |
| Glycerine | 1,5 |
| Propylenglycol | 1,5 |
| Carbomer | 0,15 |

| **Phase B** | |
|---|---|
| Glyceryl Stearate | 1,5 |
| Shea Butter fruit | 3,5 |
| Silicone-Dimethicone | 2,0 |

| **Phase C** | |
|---|---|
| Triethanolamin | 0,15 |

| **Phase D** | |
|---|---|
| Polytetrafluorethylene 10-60 µm | 8,0 |
| O₂-haltige Aggregate | |
| gemäß WO94/00098¹ | 10,0 |
| Vitamin B6 | 2,0 |
| Parfüm | 0,2 |
| Konservierungsmittel | 0,5 |

| | |
|---|---|
| ¹ gemäß Beispiel 1 der WO mit ca. 60 % Perfluorcarbon (Perfluordecalin) . | |

Die Verarbeitung erfolgte wie im Beispiel 1.

### Beispiel 7 Nacht- und Tagescreme II

| **Phase A** | |
|---|---|
| Wasser | q.s. ad 100 |
| Glycerin | 1,5 |
| Propylenglycol | 1,5 |
| Carbomer | 0,15 |

| **Phase B** | |
|---|---|
| Glyceryl Stearate | 1,5 |
| Shea Butter fruit | 3,5 |
| Silicone-Dimethicone | 2,0 |

| **Phase C** | |
|---|---|
| Triethanolamin | 0,15 |

| **Phase D** | |
|---|---|
| Polytetrafluorethylene 10-60 µm | 8,0 |
| Aggregate mit hartmagnetischem Barium- | |
| hexaferrit gemäß WO95/03061¹ | 2,0 |
| Vitamin B6 | 2,0 |
| Parfüm | 0,2 |
| Konservierungsmittel | 0,5 |

| | |
|---|---|
| ¹ gemäß Beispiel 7 mit Perfluordecalin. | |

Die Verarbeitung erfolgte wie im Beispiel 1.

## Patentansprüche

1. Kosmetische Zusammensetzung mit elektrischen Ladungsträgern, **dadurch gekennzeichnet, daß** die Zusammensetzung 0,1 bis 10 Gew-% eines kosmetisch annehmbaren Elektret-Feststoffes mit einer Teilchengröße von 0,05 bis 100 µm enthält, der als Elektret ein induziertes permanentes Dipolmoment aufweist und ein permanentes elektrischen Dipolfeld mit einer Feldstärke von 500 bis 10⁷ Vm⁻¹ hat, sowie
kosmetische Trägerstoffe, Hilfsstoffe, weitere Wirkstoffe oder ein Gemisch davon mit einem Anteil bis 100 Gew-% enthält, wobei die Prozentangaben auf das Gesamtgewicht der Zusammensetzung bezogen sind.

2. Zusammensetzung nach Anspruch 1, **dadurch gekennzeichnet, daß** der in den Elektretzustand überführte Elektret ausgewählt ist unter polymerisierten Fluorcarbonen, Polyethylenterephthalat, Polymethylmethacrylat, Polyimiden, Polypropylen, Polyethylen, Polyurethanen, Polyharnstoffen, Keramiken, Gläsern, Glaskeramiken und Gemischen davon, die jeweils in den Elektretzustand überführt worden sind.

3. Zusammensetzung nach Anspruch 2, **dadurch gekennzeichnet, daß** die polymerisierten Fluorcarbone aus der Gruppe ausgewählt sind, bestehend aus Polytetrafluorethylen (PTFE), Fluorethylenpropylen (FEP), Polyvinylidenfluorid (PVDF), amorphes Fluorpolymer (AF) und Gemische davon.

4. Zusammensetzung nach Anspruch 2, **dadurch gekennzeichnet, daß** die Keramiken oder Glaskeramiken solche mit den oxidischen Grundstoffen sind, ausgewählt aus der Gruppe, bestehend aus Zirkonoxid, Titanoxid, Magnesiumoxid, Lithiumoxid, Calciumoxid, Siliciumdioxid, Bariumoxid und Gemischen davon.

5. Zusammensetzung nach Anspruch 1, **dadurch gekennzeichnet, daß** der Elektret ein induziertes permanentes elektrisches Dipolmoment im Bereich von 10⁻¹⁵ bis 10²⁴ hat.

6. Zusammensetzung nach Anspruch 1, **dadurch gekennzeichnet, daß** der Elektret zusammen mit einem kosmetischen Wirkstoff eingesetzt wird, wobei der Wirkstoff ausgewählt ist unter einem Vitamin A-haltigen Produkt mit wenigstens 0,1-2 Gew-% Vitamin A-Anteil an der Gesamtzusammensetzung, einem Vitamin E-haltigen Produkt mit wenigstens 0,1-2 Gew-% Vitamin E-Anteil an der Gesamtzusammensetzung, einem kreatinhaltigen Produkt im Bereich von 0,1-3 Gew-% oder einem Gemisch davon.

7. Zusammensetzung nach Anspruch 6, **dadurch gekennzeichnet, daß** der Vitamin A-Anteil oder der Vitamin E-Anteil durch eine Vitamin A- oder E-Derivat gebildet wird.

8. Zusammensetzung nach Anspruch 1, **dadurch gekennzeichnet, daß** der Elektret ein permanentes elektrisches Feld mit einer Koerzitivkraft von 10⁴ bis 10⁶ Vm⁻¹ hat.

9. Verwendung von Elektreten mit einem Dipolfeld mit einer Feldstärke von 500 bis 10⁷ Vm⁻¹ in einem Anteil von 0,1 bis 10 Gew-% und mit einer Teilchengröße von 0,05 bis 100 µm, ausgewählt unter polymerisierten Fluorcarbonen, Polyethylenterephthalat, Polymethylmethacrylat, Polyimiden, Polypropylen, Polyethylen, Polyurethanen, Polyharnstoffen, Keramiken, Gläsern, Glaskeramiken und Gemischen davon, die jeweils in einen induzierten Elektretzustand überführt worden sind, zusammen mit kosmetischen Trägerstoffen, Hilfsstoffen, weiteren Wirkstoffen oder einem Gemisch davon mit einem Anteil bis 100 Gew-% in kosmetischen Cremes, Lotionen, Gelen, Pudern und Stiften zur verbesserten Aufnahme von Nährstoffen und/oder Wirkstoffen in die Haut.

10. Verwendung nach Anspruch 9 zusammen mit einem Wirkstoff, ausgewählt aus der Gruppe, bestehend aus Vitamin A, Vitamin B, Vitamin E, Kreatin, Derivaten davon und Gemischen davon.

11. Kosmetische Zusammensetzung mit elektrischen Ladungsträgern, **dadurch gekennzeichnet, dass** die Zusammensetzung 0,1 bis 10 Gew-% eines kosmetisch annehmbaren Elektret-Feststoffes mit einer Teilchengröße von 0,05 bis 100 µm enthält, der als Elektret ein induziertes permanentes Dipolmoment aufweist und ein permanentes elektrischen Dipolfeld mit einer Feldstärke von 500 bis 10⁷ Vm⁻¹ hat, sowie kosmetische Trägerstoffe, Hilfsstoffe, weitere Wirkstoffe oder ein Gemisch davon mit einem Anteil bis 100 Gew-% enthält, wobei die Prozentangaben auf das Gesamtgewicht der Zusammensetzung bezogen sind,
erhältlich durch Erhitzen eines nicht-ferromagnetischen Feststoffes auf eine Temperatur unterhalb seiner Schmelztemperatur, Aussetzen des Feststoffes einem elektrischen Feld von 1000 bis 10⁷ V/m, spontane Abkühlung des Feststoffes, Mahlen des hergestellten Elektret-Feststoffes auf eine Teilchengröße von 0,05 bis 100 µm und Einmischen in eine kosmetische Zusammensetzung unterhalb 50 °C.
